# EUROPÄISCHE PATENTSCHRIFT

(11) **EP 1 414 467 B1**
(45) Veröffentlichungstag und Bekanntmachung des Hinweises auf die Patenterteilung: **26.04.2006**
(21) Anmeldenummer: 01992575.9
(22) Anmeldetag: 30.10.2001
(51) Int. Cl.: A61K 31/5685, A61P 29/00, A61K 31/565, A61K 31/00, A61K 45/06

(54) **TOPISCHE BEHANDLUNG BEI DER MASTALGIE**
TOPICAL TREATMENT FOR MASTALGIA
TRAITEMENT TOPIQUE DE LA MASTALGIE

(30) Priorität: 02.11.2000 DE 10054294
(43) Veröffentlichungstag der Anmeldung: 06.05.2004
(73) Patentinhaber: Schmidt, Alfred, 79183 Waldkirch (DE); WIELAND, Heinrich, 79271 St. Peter (DE)
(72) Erfinder: Schmidt, Alfred, 79183 Waldkirch (DE); WIELAND, Heinrich, 79271 St. Peter (DE)
(86) Internationale Anmeldenummer: PCT/EP2001/012536
(87) Internationale Veröffentlichungsnummer: WO 2002/036129

(56) Entgegenhaltungen:
- EP-A- 0 342 665
- EP-A- 0 684 235
- WO-A-00/43553
- WO-A-01/12206
- WO-A-92/18132
- WO-A-97/36570
- WO-A-99/47143
- WO-A-99/49851
- US-A- 5 202 314
- US-A- 5 641 877
- US-A- 5 804 168
- US-A- 6 020 327

## Beschreibung

Die vorliegende Erfindung betrifft eine topische Zusammenseztung zur Behandlung der Mastalgie bzw. deren Symptome.

Die Mastalgie (auch Mastodynie genannt) ist ein bei Frauen weit verbreiteter Zustand schmerzhafter Empfindungen der Brust. Schätzungen gehen davon aus, dass etwa zwei Drittel der arbeitenden weiblichen Bevölkerung an solchen Brustschmerzen leiden. Ihr zyklisches zeitliches Auftreten läßt sich leicht in Verbindung mit dem Menstruationszyklus bringen. Sie sind sehr häufig Teil des Symptomenkomplexes "prämenstruelles Syndrom". Die Ursachen für die auftretenden Symptome scheinen komplex und von der Fachwelt bisher wenig verstanden.

Entsprechend sind die herkömmlichen Behandlungsverfahren gegen Mastalgie in Bezug auf Prinzip und Wirkungsziel sehr verschieden (s. Überblick von P.A. Holland und C.A. Gatelay in "Drugs" 48 (5), S. 709-716 (1994), Titel: "Drug therapy of mastalgia. What are the options?"). Zu den Möglichkeiten gehören z.B. die Anwendung von Analgetika oder Diuretika. Andererseits werden häufig Naturprodukte wie Nachtkerzenöl- und Vitamin B6-Präparate angewandt, denen jedoch keine oder kaum über einen Placeboeffekt hinausgehende Wirkungen zugeschrieben werden. Andere Ansätze verfolgen einen systemischen Eingriff in den Stoffwechsel, insbesondere durch systemische Applikation des Prolaktinhemmers Bromocriptin, des Gonadotropinhemmers Danazol und des Antiöstrogens Tamoxifen. Diese systemischen Anwendungen, vor allem mit dem Zytostatikum Tamoxifen, sind jedoch mit beträchtlichen Nebenwirkungen verbunden und wird trotz der nachgewiesenen Wirkung von ärtztlicher Seite nur in besonders persistenten Fällen angeraten. Zur Reduzierung der Nebenwirkungen ist eine Therapiekontrolle mit der Verabreicherung niedriger Dosen angezeigt. Auch der chirurgische Eingriff zur lokalen Biopsie steht im weiten Spektrum der möglichen Behandlung. Gegen solche chirurgischen Eingriffe gibt es von fachärtztlicher Seite jedoch ebenfalls starke Einwände.

Aufgabe der vorliegenden Erfindung war es daher, die Behandlungsmöglichkeiten von Mastalgie zu verbessern.

Die Aufgabe wird gelöst durch die

Verwendung eines steroidalen Aromatase-Inhibitors oder einer diesen enthaltenden Zusammensetzung zur Prophylaxe und/oder Behandlung gegen Mastalgie mittels topischer Applikation. Die erfindungsgemäße Verwendung zielt auf die Beseitigung der Brustschmerzsymptome ab und schließt die Herstellung eines pharmazeutischen Erzeugnisses ein.

Bevorzugte Ausführungsformen der Erfindung sind in den Unteransprüchen zu Anspruch 1 gekennzeichnet. Die Erfindung ist auf besonders geeignete Weise einsetzbar bei lokal topischer Applikation auf den Brustbereich der weiblichen Brust, insbesondere der schmerzhaften Zonen. So kann auf vorteilhafte Weise ein Mastalgie lindernder Effekt gezielt und selektiv erreicht werden, ohne jedoch das physiologische Gleichgewicht mit dem normalen Sexualhormon-Körperhaushalt im unbehandelten, also weitaus größten Körperbereich merklich zu beeinträchtigen.

Der erfindungsgemäße Behandlungsansatz folgt einem völlig neuen Konzept und unterscheidet sich grundsätzlich von herkömmlichen Behandlungsansätzen. Während herkömmliche Behandlungen vornehmlich auf systemischen Medikationssystemen oder gelegentlich auf operativchirurgischen Maßnahmen beruhen und mit starken Nebenwirkungen oder anderen Nachteilen behaftet sind, geht die Erfindung einen anderen Weg und greift wirksam und lokal in die extragonadalen Synthesewege von Sexualsteroiden ein. Weil die Applikation nicht systemisch, sondern lokal topisch erfolgt und ein zentrales Enzym in der extragonadalen Biosynthese im peripheren, behandelten Körperbereich hemmt, ist die durch die Erfindung zur Verfügung gestellte Behandlung sehr nebenwirkungsarm und gut verträglich. Die lokal topische Applikation ist daher nicht nur zur Therapie in der akuten Situation der Schmerzempfindung, sondern auch als prophylaktische Maßnahme sehr nützlich.

Der Mechanismus der erfindungsgemäßen, lindernden Wirkung bei der Mastalgie ist nicht klar. Zum einen kann ein primärer Effekt vermutet werden, der wegen der Verminderung der lokalen Östrogensynthese auf der Erzeugung eines Mangels an lokal im Körper (z.B. in der Haut bzw. den Drüsen und Hautanhangsgebilden) verfügbaren Östrogenen wie Östradiol, Östron und Östrol beruhen kann. Zum anderen ist auch ein Sekundäreffekt denkbar, möglicherweise weil Brustfettzellen wegen des Mangels an lokalen Östrogenen schrumpfen und so weniger Spannung oder Druck aufbauen können. Möglicherweise ergänzen sich auch Primär- und Sekundäreffekte und wirken der für die Mastalgie typischen Überempfindlichkeit entgegen. Den Wirkmechanismen zugrunde liegt jedenfalls ein lokaler Effekt in der weiblichen Brust, da die Wirksubstanz aufgrund der topischen Applikation ohne Umweg über den Blutkreislauf direkt dort hin gelangt.

Der als Wirksubstanz eingesetzte Inhibitor der (Cytochrom-p450)-Aromatase hemmt lokal die extragonadale Östrogen-Bildung. Erfindungsgemäß können einzelne oder gleichzeitig verschiedene Aromataseinhibitor-Verbindungen als Wirkstoffe in einer Zusammensetzung verwendet werden.

Beispiele für steroidale Aromatase-Inhibitoren schließen folgende Substanzen ein:
4-Hydroxyandrost-4-en-3,17-dion (Formestan und Lentaron),
6-Methylenandrostra-1,4-dien-3,17-dion (Exemestan),
10-(2-Propynyl)estr-4-en-3,17-dion (MDL 18962)
7 alpha substituierte Androstendion-Derivate
1,4,6-androstatriene-3,17-dion (ATD)
10-Oxiran- und 10-Thiirane substituierte Androgene
10-Propargylestr-4-ene-3,17-dione
10-propargylestr-4-ene-3,17-propionate 10-(2-propynyl)-Derivat
13-retro-Antiprogestine
14 alpha-hydroxy-4-androstene-3,6,17-trione (14 alpha-OHAT)
16- oder 19-substituierte Androst-4-ene
19-(Cyclopropylamino)-androst-4-en-3,17-dion
19-(Ethyldithio)-androst-4-ene-3,17-dione (ORG 30958)
19-Oxiranyl- and 19-Thiiranyl-Steroide
19-Thiomethyl- and 19-Azido-androstenedion
1-Methyl-androsta-1,4-diene-3,17-dione (Atamestan)
2,2-Dimethyl-4-hydroxy-4-androstene-3,17-dion
3 alpha-methoxyandrost-4-ene-6,17-dione
3 beta-hydroxyandrost-4-en-6-one-Derivate
3-Deoxyandrogen-19-Oxygenierderivate von 3-oxo-17 betacarboxamido-Steroide
4-(Phenylthio)-4-androstene-3,17-dion
4-(Thio-substituiertes)-4-androstene-3,17-dion
4-Acetoxy-4-androsten-3,17-dion
4-Aminoandrostenedion
4-Androstene-3,6,17-trion
4-Hydroxyandrostenedion (4-OHA, CGP 32349)
4-Methoxy-4-androstene-3,17-dion
4-Oxygenierte Androst-5-en-17-one und deren 7-oxo-Derivate
4-Thiosubstituierte Derivate von 4-Androsten-3,17-dion
4-Thiosubstituierte-4-androsten-3,17-dion-Derivate
5 alpha-Dihydronorethindron (ein Metabolit von Norethindron)
5 alpha-reduzierte C19-Steroide
5 alpha-Androstan-17-ones mit oder ohne eine Carbonylfunktion an C-3 und/oder C-6
6 alpha,7 alpha-Cyclopropanderivate von Androst-4-en
6 alpha-Fluorotestosteron
6 beta-Propynyl-substituierte Steroide
6,7-Aziridinylsteroid und verwandte Verbindungen
6-Alkylanaloge von delta 1,4,6-Androgenen
6-Alkylanaloge of delta 4,6-Androgenen
6-Alkyl- und 6-Arylandrost-4-en-3,17-dione
6-Alkylandrost-4-en-3,17-dione von 7 alpha- and 7 beta-arylaliphatisch-substituierten Androst-4-en-3,17-dionen
6-Alkylandrosta-4,6-dien-3,17-dione und deren 1,4,6-trien-Analoga
6-Alkyl-substituierte Androgene
6-Phenylaliphatisch-substituierte C19-Steroide mit 1,4-dien-, 4,6-dien- oder 1,4,6-trien-Struktur
6-Bromoandrostenedion
6-Hydroximinoandrostenedion
6-Methylenandrosta-1,4-dien-3,17-dion (FCE 24304)
6-Methylenandrosta-1,4-dien-3,17-dion (FCE 24304)
6-Phenylaliphatisch-substituierte Androst-4-en-3,17-dione
6-substituierte Androst-4-en-Analoga
7 alpha-(4'-Amino)phenylthio-4-androsten-3,17-dion
7 alpha-substituierte Androsta-1,4-dien-3,17-dione
7 alpha-substituierte Androstenedione
7 alpha-(4'-Amino)phenylthio-4-androsten-3,17-dion
7 alpha-arylaliphatische Androsta-1,4-dien-3,17-dione
7 alpha-substituierte Androstenedione
7 substituierte 4,6-Androstadien-3,17-dione
7 substituierte Steroide
Androst-4-en-3,6-dionderivative
Androst-5-ene-7,17-dion 19-nor- und 5 beta,6 beta-epoxy-Derivative
A-or B-ring-substituierte Derivative von Androst-4-en-3,6,17-trion
A-ring verbrückte Steroid
Bromoacetoxy 4-androsten-3-one
delta 1,4,6-Androgene
delta 4,6-Androgene
epimere 6-Hydroperoxyandrostendione
estr-4-ene-3, 17-dion (MDL 18 962),
estr-4-ene-3,6,17-trione
Flavonoide
RU486

Hinsichtlich der Bezeichnungen dieser Substanzen sowie deren Verfügbarkeit siehe beispielsweise "Rote Liste", Editio Cantor, Aulendorf (DE), (1999).

Solche Aromatase-Inhibitoren sind an sich bekannt, hauptsächlich als systemisch eingesetzte Wirkstoffe zur medizinisch-therapeutischen Behandlung von Brustkrebs. In diesem Zusammenhang wird verwiesen auf die Übersichtsartikel von A.M.H. Brodi in: "J. Steorid Biochem. Molec. Biol.", Vol. 49, No. 4-6, pp. 281-287 (1994), A.M.H. Brodi in "Biochemical Pharmacology", Vol. 34, No. 18, pp. 3213-3219 (1985) sowie P. E. Goss und K.M.E.H. Gwyn in: "Journal of Clinical Oncology", Vol. 12, No. 11, pp. 2460-2470 (1994). Zur Bestimmung der Aromatase-Inhibition und der nachfolgenden Östrogenreduzierung wird auf die in den genannten Übersichtsartikeln angegebenen, weiteren Literaturnachweise verwiesen, s. beispielsweise A.M.H. Brodi et al. in: "J. Steroid Biochem. Molec. Biol.", Vol. 7, pp. 787-793 (1976), und D.A. Marsh et al. in: "J. Med. Chem.", Vol. 28, pp. 788-795 (1985).
In der WO-A-96/08231 werden Aromatase-Inhibitoren zur Bekämpfung des Haarausfalls verwendet. In einem Medikament zur Beeinflussung des Haarwachstums liegt neben dem Aromataseinhibitor ein Androgenrezeptor-Antagonist vor. Die Förderung des Haarwachstums ist jedoch eine von der Mastalgie grundsätzlich verschiedene Problemstellung, und ein in der Druckschrift bezeichneter Androgenrezeptor-Antagonist ist im Rahmen der vorliegenden Erfindung nicht erforderlich.
Spezielle Azolderivate und deren aromatasehemmende und antimycotische Wirkung werden ferner beschrieben in der EP-A-0 575 210.
Das US-Patent Nr. 4 937 250 offenbart spezielle Verbindungen mit Nitril- und Imididazolyl- und ggf. weiter substitiertem Toluolgrundgerüst (alpha-Heterocyclus substituierte Tolunitrile) als aromatasehemmende Wirkstoffe und schlägt die jeweiligen Wirkstoffe enthaltende, pharmazeutische Zusammensetzungen zur oralen, rectalen, transdermalen oder parenteralen Applikation gegen Östrogensynthese abhängige Zustände wie Gynecomastia, Mamma- und endometrialen Tumoren, Endometriosis und Frühwehen vor.

In dem US-Patent Nr. 4 895 715 wird eine Behandlung gegen Gynecomastia beim Mann, speziell gegen eine benigne Form der Hypertrophie der Prostata vorgeschlagen, bei der ein Antiandrogen (z.B. Flutamid) zusammen mit einem Aroamtseinhibitor (4-Hydroxyandrosteron) oder mit einem Antiöstrogen (Tamoxifen) oral oder parenteral, d.h. systemisch, verabreicht wird.
Für weitere spezielle Anwendungen sind Aromataseinhibitoren als Substanzen beschrieben worden, die sich günstig zur kosmetischen Behandlung von gestörtem Unterhaut-Bindefettgewebe, insbesondere der Cellulite (s. WO-A-97/36570), sowie zur Straffung und Verkleinerung von Fettzellen-haltigen Körperpartien, insbesondere der Bruststraffung (s. WO-A-99/17712), auswirken. Aus solchen Anwendungsfällen können jedoch keine Rückschlüsse gezogen werden für das Problem der Behandlung der Mastalgie. Ein Mastalgie lindernder Effekt war nicht zu erwarten.

Die EP-A-0 684 235 bezieht sich auf einen Aromatase-Inhibitor (Azolyl-Methyl-Phenyl-Derivate) im Hinblick auf verschiedene östrogenabhängige Krankheiten, u.a. die Mastopathie.

Die WO 99/49851 A beschreibt im Abschnitt 5.3 eine Chrysinenthaltende Formulierung, die angeblich eine Hemmung der Aromatase ermöglichen soll, als Diätformulierung zur Konditionierung und zum Aufbau der Muskelmasse von Athleten. Im Abschnitt 5.5 wird Grapefruitsaft allein oder in Kombination mit DIM (3,3'-Diindolylmethan) in einer Formulierung, die kein Chrysin enthält, zur Behandlung von Mastalgie beschrieben. In der WO 99/49851 A geht es generell um die Unterstützung der Absorption von hydrophoben Diätverbindungen, so dass also die in diesem Dokument beschriebenen Formulierungen oral verabreicht werden müssen.

In der WO 99/47143 A geht es um die Verwendung eines steroidalen Aromatase-Inhibitors zur Prophylaxe und/oder Behandlung des Mammakarzinoms mittels topischer Applikation.

Eine erst in der WO-A-97/36570 erschlossene, neue Substanzwahl innerhalb der Klasse der Aromataseinhibitoren, nämlich solcher aromatasehemmender Sterole (Glycine), die aus Soja stammen (Soja-Gycinen, INCI-Name nach dem Linne-System), können auch im Rahmen der vorliegenden Erfindung eingesetzt werden. Insoweit wird auf die WO-A-97/36570 verwiesen. Dies gilt auch für die dort ebenfalls beschriebenen oxidativ behandelten Soja-Glycine.

In Kombination mit den Aromataseinhibitoren kann in der topischen Zusammensetzung, um die Mastalgie lindernde Wirkung zu steigern, eine zur Gruppe der Anti-Östrogene gehörende Substanz eingeschlossen sein. Als Beispiele für Substanzen der Anti-Östrogenklasse sind insbesondere die nicht-steroidalen Östrogen-Antagonisten Tamoxifen (Z-2-[4-(1,2-Diphenyl-1-butenyl)-phenoxy]-N,N- dimethylamin) und Aminoglutethimid (3-(4-Aminophenyl)-3-ethyl-2,6-piperidin-dion) sowie deren Analoga und Derivate, beispielsweise das 3-Hydroxytamoxifen, das 4-Hydroxytamoxifen sowie das 7 α-Alkyl-Sulfinyl-Tamoxifen-Analoge (ICI 182,780), zu erwähnen. Diese erfindungsgemäße Kombinationszusammensetzung ist vorteilhaft aufgrund der besseren Verträglichkeit der topischen Applikation im Gegensatz zur systemischen Applikation von z.B. Tamoxifen mit den damit verbundenen Nebenwirkungen. Es können auch ein oder mehrere steroidalen Aromatase-Inhibitoren mit einem oder mehreren Anti-Östrogenen kombiniert werden. Das bei der Kombination zu verwendende, gewichtsmäßige Mengenverhältnis von steroidalen Aromatase-Inhibitor zu Anti-Östrogen liegt geeigneterweise in einem Bereich von 90/10 bis 10/90, insbesondere in einem Bereich von 60/40 bis 40/60.

Um den therapeutischen Ansatz der vorliegenden Erfindung noch gezielter zu ermöglichen und besser abstimmen zu können, kann eine oder mehrere der oben beschriebenen Aromataseinhibitoren und ggf. Antiöstrogene mit einem weiteren Wirkprinzip derart kombiniert werden, dass zusätzlich oder gleichzeitig die Bildung und/oder die Wirkung von Dihydrotestosteron gehemmt wird. Ein besonderer Vorteil der vorliegenden Erfindung ist dadurch begründet, dass manche der oben genannten steroidalen Aromatase-inhibitoren den erwünschten Effekt der gleichzeitigen Hemmung des Enzyms 5-alpha-Reduktase besitzen, das für die Reduktion des Testosterons zum wirksamen Androgen Dihydrotestosteron (DHT) verantwortlich ist. Ohne die gleichzeitige Hemmung der 5-alpha-Reduktase überwiegen nach Hemmung allein der Aromatase in der Brust und in der Brusthaut Androgene, was zu lokalen Vermännlichungserscheinungen wie Haarwuchs oder Akne führen könnte. Die Hemmung der 5-alpha-Reduktase kann nicht nur durch Einsatz solcher Substanzen mit gleichzeitiger Aromatase- und 5-alpha-Reduktase-Hemmwirkung, sondern auch durch kombinierte topische Applikation eines Aromatasehemmers ohne die Hemmwirkung auf die 5-alpha-Reduktase mit Hemmstoffen der 5-alpha-Reduktase erreicht werden.

Beispiele für 5-alpha-Reduktase-Inhibitoren schließen ein, wobei je nach Typ klassifiziert wird:
Typ 1-Hemmer:
   LY191704 (Benzochinolinon)
   4,7 beta-Dimethyl-4-azacholestan-3-on (MK-386) und verwandte 4-Azasteroide
   Benzo [c] chinolizin-3-on
Typ 2-Hemmer:
   Benzophenon- and Indolcarboxylsäuren
   N-tert-Butyl-3-oxo-4-aza-5a-androst-1-en-17-(3-carboxamid (Finasterid)
Duale Hemmer (Typ 1 und Typ 2):
   3-Carboxy-20-keto-Steroide
   6-Azasteroid
   4-Aza-3-oxo-5 alpha-androst-1-en-17 beta-N-aryl-6-Azasteroide
   FK143
Nichtsteroidale Hemmer:
   4-(1-Benzoylindol-3-yl)buttersäuren
   4-[3-[3-[Bis(4-isobutylphenyl)methylamino]benzoyl]-1H-indol-1-yl]-buttersäure
   Benzanilid-Derivate
   Carbamoylalkenyl)phenyloxy carboxylsäure-Derivate
   Ethyl-4-(1-methyl-2-oxopiperid-5-yl)benzoat
   FK143
   N,N-bis(1-Methylethyl)-4-[3-(1,2-dihydro-1-methyl-2-oxopyrid-5-yl)propyl]benzamid
   Phenoxybenzoesäure-Derivate
   Carboxamid- und Phenylalkyl-substituierte Pyridone und Piperidone
   Natrium-4-[2-(2,3-dimethyl-4-[1-(4-isobutylphenylethoxy] benzolamino)phenoxy]butyrat (ONO-3805)
   (Z)-4-2-[[3-[1-(4,4'-Difluorobenzhydryl) indol-5-yl]-2-pentenoyl]-amino]phenoxy]butyric acid (KF20405)
Steroidale Hemmer:
   17 beta-(N,N-Diisopropylcarbamoyl)estra-1,3,5(10)-trien-3-sulfonsäure
   17 beta-Carbamoyl-1,3,5(10)-estratrien-3-carboxylsäuren
   17 beta-N,N-Diethylcarbamoyl-4-methyl-4-aza-5 alpha-androstan-3-on (4-MA)
   17 beta-N-(2-Methyl-2-propyl)-carbamoyl-androst-3,5-dien-3-carboxylsäure
   3-Androsten-3-carboxylsäuren (steroidale Acrylate),
   3-Carboxy-17 beta-substituierte Steroid
   4-aza-3-oxo-Steroidfamilie
   4-Hydroxy-androstenedion
   4-Methyl-4-aza-5 alpha-pregnan-3-on-20(S)-carboxylat
   6-Methylen-progesteron-, -androsten- und -androstan-Derivate
   Finasterid
   Progesteron
   Natrium-4-methyl-3-oxo-4-aza-5 alpha-pregnan-20 (S)-carboxylat
   Steroidale A-Ring-Arylcarboxylsäuren
   TZP-4238 (steroidales Antiandrogen)

Auch diese Substanzen zur Hemmung der Bildung und/oder Wirkung von Dihydrotestosteron sind an sich bekannt, allerdings lediglich zur Behandlung benigner Prostata-Hyperplasie (s. "Rote Liste", Editio Cantor, Aulendorf (DE), (1999)).

Als Beispiel für Substanzen mit bifunktioneller Fähigkeit, d.h. die sowohl eine Aromatse- als auch eine 5-alpha-Reduktase-Hemmwirkung entfalten können, ist das dem Androstendion sehr ähnliche Sterol 4-Hydroxyandrostendion sowie deren Derivate, z.B. das o.g. Formestan, oder die Sojasterole zu nennen.

Im Sinne einer gesteigerten Effizienz sind erfindungsgemäß solche steroidalen Aromatase inhibitoren und ggf. solche Antiöstrogen und/oder 5-alpha-Reduktaseinhibitoren bevorzugt, die einen lipophilen Charakter aufweisen. Solche lipophile Substanzen, insbesondere wenn sie in ein geeeignetes pharmakologisches Hautpermeations-Vehikelsystem eingebracht sind, können leicht durch die Hautschichten sowie Zellmembranen passieren und somit schnell und effizient ihre Wirkung entfalten. Solche Substanzen bzw. die diese Substanzen enthaltenden Zusammensetzungen sind daher besonders gut für die topische Anwendung geeignet. Dies trifft insbesondere auf die steroidalen Aromatase-Inhibitoren zu, die von Natur aus durch das Steroid-Gerüst einen lipophilen Charakter besitzen und in der Regel eine gute bis sehr gute perkutane Resorptionsfähigkeit und Zellmembrangängigkeit aufweisen. Zudem können sich die lipophilen Substanzen leicht im Fettgewebe anreichern. Substanzen, die von Natur aus eine erwünschte, hohe Lipophilie nicht aufweisen, können auf an sich bekannte Weise durch Modifizierung bzw. Derivatisierung mit lipohilen Gruppen lipophil gemacht werden, ohne die Mastalgie lindernde Wirkung zu verlieren.

Falls in einzelnen Fällen die perkutane Resorption Probleme bereitet, oder falls eine gesteigerte perkutane Resorption erreicht werden soll, können in der verwendeten Zusammensetzung vorzugsweise zusätzlich Mittel zur Förderung der perkutanen Resorption eingesetzt werden. Solche Mittel zur Förderung der perkutanen Resorption sind bekannt. Beispielsweise eignen sich hierfür Hyaluronidate, Dimethylsulfoxid (DMSO) und dergleichen.

Zur topischen Anwendung kann eine hierfür geeignete Formulierung des zu verwendenden Stoffs gewählt werden, z.B. eine Salbe, eine Creme, ein Gel, eine Emulsion (Lotio), ein Puder, ein Öl usw.. Zu diesem Zweck umfaßt die Zusammensetzung Zusatzstoffe, die für die entsprechende Formulierung als Salbe, Creme, Gel, Emulsion, Puder oder Öl usw. üblich sind. Beschriebene sowie handelsübliche, herkömmliche Hautpflegemittel sind in den jeweiligen Formulierungen zum Einsatz in der vorliegenden Erfindung geeignet. Als übliche Zusatzstoffe für solche Formulierungen dienen beispielsweise pflanzliche Öle wie Mandelöl, Olivenöl, Pfirsichkernöl, Erdnußöl, Ricinusöl u. dergl., Pflanzenextrakte, etherische Öle, Vitaminöle, Fette und fettähnliche Stoffe, Lipoide, Phosphatide, Kohlenwasserstoffe wie Paraffine, Vaseline, Lanolin, Wachse u. dergl., Detergentien, weitere Hautwirkstoffe wie Lecithin, Wollfettalkohole, Carotin u. dergl., Hautnährstoffe, Parfums, kosmetische Stoffe, Alkohole, Glycerol, Glykole, Harnstoff, Talk, Konservierungsmittel, Sonnenschutzmittel, Farbstoffe wie Titanweiß und Zinkweiß, Antioxidantien usw.. Als Grundsubstanz dient im allgemeinen Wasser. Unter Zusatz von Emulgatoren wie Fettalkoholsulfate, Alkaliseifen, Lecitine, Triethanolamin u. dergl. kann eine O/W- oder W/O-Emulsion erhalten werden.

Sehr gut einsetzbar sind auch sogenannte transdermaltherapeutische Systeme (TTS) für die Haut, bei dem der Wirkstoff über ein klebefähiges Trägersystem, beispielsweise ein Pflaster, kontinuierlich über einen längeren Zeitraum in geeigneten Dosen auf die Haut appliziert werden kann.

Der Aromataseinhibitor kann in der Zusammensetzung in einer Menge eingesetzt werden, dass die Mastalgiesymptome wirksam eingedämmt bzw. gelindert werden. Dies kann auch in Abhängigkeit vom Schweregrad der Mastalgie bestimmt und auf den jeweiligen Anwendungsfall angepaßt werden. Geeignet ist beispielsweise ein Wirkstoffgehalt in der gesamten Zusammensetzung von 0,0001 bis 10 Gewichtsprozent (Gew.%), vorzugsweise 0,001 bis 5 Gew.% und insbesondere 0,3 bis 2 Gew.%. Für die wahlweise möglichen Antiöstrogene und/oder 5-alpha-Reduktaseinhibitoren sind entsprechende Mengenbereiche ebenfalls geeignet.

Der Gehalt des ggf. einzusetzenden Resorptionsfördermittels hängt in erster Linie von der Art des Resorptionsfördermittels ab. Die jeweils herkömmlich eingesetzten Gehaltswerte sind dabei völlig geeignet. Hyaluronidate beispielsweise können in einer Konzentration von 0,01 bis 1 Gew.%, insbesondere 0,05 bis 0,2 Gew.% verwendet werden. Für DMSO ist ein weiterer Gehaltsbereich geeignet, beispielsweise 1 bis 25 Gew.%, insbesondere 5 bis 10 Gew.%.

Die weiteren, gegebenenfalls vorhandenen Zusatzstoffe können in den für die jeweiligen Formulierungen üblichen Mengen eingesetzt werden.

Nachfolgend werden Beispiele zur weiteren Erläuterung der Erfindung beschrieben.
Es werden Behandlungen stellvertretend mit 4-Hydroxy-Androstendion beim Mastalgie - Syndrom beschrieben, wobei anstelle von 4-Hydroxy-Androstendion andere Aromatase-Inhibitoren eingesetzt werden können.

### Beispiel 1

### I. L. weiblich 17 Jahre

Befund: 3 bis 5 Tage vor jeder Menstruation auftretende spannungsbedingte Schmerzen in beiden Brüsten, scheinbar gebündelt in die Brustwarzen schießend. Während der Menstruationsperiode dann langsam abnehmende Schmerzen, Symptomatik und Spannungszustände bis etwa zum 10. Tag nach Eintritt der Menstruation.

Es wurde eine mit 0,5 Gew.-% 4-Hydroxy-Androstendion versetzte Creme eingesetzt (0,5% 4-OH-Androstendion eingebracht in 100 ml Asche-Basis-Creme). Verabreicht wurden 4g täglich, und zwar 2g äußerlich auf jede Brust aufgetragen und vorsichtig einmassiert.

Beginn des Heilversuchs zirka 3 Wochen vor der Periode. Ergebnis der ersten Folgeperiode:
Deutlich geminderter Spannungsschmerz; Symptomatik lässt schneller nach.
Bereits 5 Tage nach Menstruationsbeginn schmerzfrei.

Weiterführung des Heilversuches mit täglicher Applikation der 4-OH-Androstendion-Creme 0,5%ig..
Ergebnis der zweiten Folgeperiode: völlige Schmerzfreiheit

### Beispiel 2

### M.D., weiblich 34 Jahre

Befund: Seit Jahren jeweils 3 - 5 Tage vor der Menstruation auftretendes starkes Schwellungs- und Spannungsgefühl mit diffuser in den Brustkorb ausstrahlender Schmerzsymptomatik, die bis zum Eintritt der Menstruation schier unerträglich wird. Seit Jahren 1 - 3 Tage pro Monat wegen dieser Beschwerden arbeitsunfähig. Abklingen der Symptomatik erst am 8. - 12. Tag nach Menstruationseintritt.

Heilversuch mit 1%iger 4-OH-Androstendion-Creme (1g 4-OH-Androstendion eingebracht in 100 ml Asche-Basis-Creme).

Dosierung 1 x täglich 4g, jeweils 2g aufgetragen auf jede Brust.
Der Start des Heilversuches erfolgte zirka 2 Wochen vor Eintritt der Menstruation.

Folge - Periode 1: etwas blanderer Verlauf; 1 Tag arbeitsunfähig.

Folge - Periode 2: Deutlich geringeres Schwellungs- und Spannungsgefühl; erheblich geringere Schmerzsymptomatik; nur noch in Brüsten lokalisiert, keine Arbeitsunfähigkeit.

Fortführung des Heilversuches mit 0,5%iger 4-OH-Androstendion-Creme (0,5 g 4-OH-Androstendion eingebracht in 100 ml Asche-Basis-Creme) 4g täglich, d.h. 2 g auf jede Brust aufgetragen.

Folgeperioden: Kaum noch Schwellungs- und Spannungsgefühle; praktisch schmerzfrei. Seither keine Mastopathie - bedingte Arbeitsunfähigkeit.

## Patentansprüche

1. Verwendung eines steroidalen Aromatase-Inhibitors oder einer einen steroidalen Aromatase-Inhibitor enthaltenden Zusammensetzung zur Herstellung eines Medikaments zur Prophylaxe und/oder Behandlung von Mastalgie mittels topischer Applikation.

2. Verwendung nach Anspruch 1, **dadurch gekennzeichnet, daß** der steroidale Aromatase-Inhibitor einen lipophilen Charakter aufweist.

3. Verwendung nach Anspruch 2, **dadurch gekennzeichnet, dass** der steroidale Aromatase-Inhibitor ein aromatasehemmendes Sterol ist.

4. Verwendung nach Anspruch 3, **dadurch gekennzeichnet, dass** das aromatasehemmendes Sterol aus Soja-Glycine stammt.

5. Verwendung nach einem der vorangehenden Ansprüche, **dadurch gekennzeichnet, dass** der steroidale Aromatase-Inhibitor in einer Zusammensetzung in einer Menge von 0,001 bis 5 Gew.%, bezogen auf die Gesamtmenge der Zusammensetzung, enthalten ist.

6. Verwendung nach einem der vorangehenden Ansprüche, **dadurch gekennzeichnet, dass** die Zusammensetzung ferner ein Anti-Östrogen enthält.

7. Verwendung nach einem der vorangehenden Ansprüche, **dadurch gekennzeichnet, dass** als steroidaler Aromatase-Inhibitor eine Substanz dient, die gleichzeitig eine Hemmwirkung gegenüber der 5-alpha-Reduktase besitzt.

8. Verwendung nach einem der Ansprüche 1 bis 6, **dadurch gekennzeichnet, dass** die Zusammensetzung ferner eine vom eingesetzten steroidalen Aromatase-Inhibitor verschiedene Substanz enthält, die eine 5-alpha-Reduktase-Inhibitorwirkung besitzt.

9. Verwendung nach einem der vorangehenden Ansprüche, **dadurch gekennzeichnet, dass** die Zusammensetzung ferner Mittel zur Förderung der perkutanen Resorption umfaßt.

10. Verwendung nach einem der vorangehenden Ansprüche, **dadurch gekennzeichnet, dass** die Zusammensetzung als Salbe, Creme, Gel, Öl oder Emulsion bzw. Lotio formuliert ist.

11. Verwendung nach Anspruch 10, **dadurch gekennzeichnet, dass** die Zusammensetzung Zusatzstoffe umfaßt, die für die entsprechende Formulierung als Salbe, Creme, Gel, Öl, Emulsion bzw. Lotio üblich sind.

12. Verwendung nach einem der vorangehenden Ansprüche, **dadurch gekennzeichnet, dass** die Zusammensetzung in einem Transdermalsystem integriert ist.

13. Verwendung nach einem der vorangehenden Ansprüche, **dadurch gekennzeichnet, dass** die topische Applikation zur lokalen Applikation auf der weiblichen Brust dient.

## Claims

1. Use of a steroidal aromatase inhibitor or of a composition comprising a steroidal aromatase inhibitor for producing a medicament for the prophylaxis and/or treatment of mastalgia by topical application.

2. Use according to Claim 1, **characterized in that** the steroidal aromatase inhibitor has a lipophilic character.

3. Use according to Claim 2, **characterized in that** the steroidal aromatase inhibitor is an aromatase-inhibiting sterol.

4. Use according to Claim 3, **characterized in that** the aromatase-inhibiting sterol is derived from soya glycine.

5. Use according to any of the preceding claims, **characterized in that** the steroidal aromatase inhibitor is present in a composition in an amount of from 0.001 to 5% by weight, based on the total amount of the composition.

6. Use according to any of the preceding claims, **characterized in that** the composition additionally comprises an anti-oestrogen.

7. Use according to any of the preceding claims, **characterized in that** the substance serving as steroidal aromatase inhibitor simultaneously has an inhibitory effect on 5-alpha-reductase.

8. Use according to any of Claims 1 to 6, **characterized in that** the composition additionally comprises a substance which is different from the steroidal aromatase inhibitor employed and which has a 5-alpha-reductase-inhibitory effect.

9. Use according to any of the preceding claims, **characterized in that** the composition additionally comprises means for promoting percutaneous absorption.

10. Use according to any of the preceding claims, **characterized in that** the composition is formulated as ointment, cream, gel, oil or emulsion, or lotion.

11. Use according to Claim 10, **characterized in that** the composition includes additives which are customary for the corresponding formulation as ointment, cream, gel, oil, emulsion, or lotion.

12. Use according to any of the preceding claims, **characterized in that** the composition is integrated in a transdermal system.

13. Use according to any of the preceding claims, **characterized in that** the topical application serves for local application to the female breast.

## Revendications

1. Utilisation d'un inhibiteur stéroïdien d'aromatase ou d'une composition contenant un inhibiteur stéroïdien d'aromatase, pour la fabrication d'un médicament destiné à la prophylaxie et/ou au traitement de la mastalgie par application topique.

2. Utilisation selon la revendication 1, **caractérisée en ce que** l'inhibiteur stéroïdien d'aromatase a un caractère lipophile.

3. Utilisation selon la revendication 2, **caractérisée en ce que** l'inhibiteur stéroïdien d'aromatase est un stérol inhibiteur d'aromatase.

4. Utilisation selon la revendication 3, **caractérisée en ce que** le stérol inhibiteur d'aromatase est dérivé de glycines de soja.

5. Utilisation selon l'une quelconque des revendications précédentes, **caractérisée en ce que** l'inhibiteur stéroïdien d'aromatase est contenu dans une composition en une quantité de 0,001 à 5 % en poids, par rapport à la quantité totale de la composition.

6. Utilisation selon l'une quelconque des revendications précédentes, **caractérisée en ce que** la composition contient en outre un anti-oestrogène.

7. Utilisation selon l'une quelconque des revendications précédentes, **caractérisée en ce qu'**on utilise en tant qu'inhibiteur stéroïdien d'aromatase une substance qui a en même temps une activité inhibitrice vis-à-vis de la 5-alpha-réductase.

8. Utilisation selon l'une des revendications 1 à 6, **caractérisée en ce que** la composition contient en outre une substance différente de l'inhibiteur stéroïdien d'aromatase utilisé, qui a une activité inhibitrice vis-à-vis de la 5-alpha-réductase.

9. Utilisation selon l'une quelconque des revendications précédentes, **caractérisée en ce que** la composition comprend en outre des agents destinés à favoriser la résorption percutanée.

10. Utilisation selon l'une quelconque des revendications précédentes, **caractérisée en ce que** la composition est formulée sous forme d'une pommade, d'une crème, d'un gel, d'une huile ou d'une émulsion ou d'une lotion.

11. Utilisation selon la revendication 10, **caractérisée en ce que** la composition comprend des additifs, qui sont habituellement utilisés pour la formulation correspondante sous forme d'une pommade, d'une crème, d'un gel, d'une huile, d'une émulsion ou d'une lotion.

12. Utilisation selon l'une quelconque des revendications précédentes, **caractérisée en ce que** la composition est intégrée dans un système transdermique.

13. Utilisation selon l'une quelconque des revendications précédentes, **caractérisée en ce que** l'application topique sert à l'application locale sur le sein féminin.
